(19) Europäisches Patentamt
European Patent Office
Office européen des brevets

(11) **EP 1 254 106 B1**

(12) **EUROPEAN PATENT SPECIFICATION**

(45) Date of publication and mention of the grant of the patent:
**25.02.2009 Bulletin 2009/09**

(21) Application number: **01910349.8**

(22) Date of filing: **24.01.2001**

(51) Int Cl.:
***C07C 255/50*** *(2006.01)* ***A61K 31/277*** *(2006.01)*

(86) International application number:
**PCT/US2001/002402**

(87) International publication number:
**WO 2001/053254 (26.07.2001 Gazette 2001/30)**

(54) **CALCILYTIC COMPOUNDS**

CALCILYTISCHE VERBINDUNGEN

COMPOSES CALCILYTIQUES

(84) Designated Contracting States:
**AT BE CH CY DE DK ES FI FR GB GR IE IT LI LU MC NL PT SE TR**
Designated Extension States:
**RO SI**

(30) Priority: **24.01.2000 US 177683 P**

(43) Date of publication of application:
**06.11.2002 Bulletin 2002/45**

(73) Proprietors:
- **SMITHKLINE BEECHAM CORPORATION**
  **Philadelphia, PA 19103 (US)**
- **NPS Pharmaceuticals, Inc.**
  **Bedminster NJ 07921 (US)**

(72) Inventors:
- **LAGO, Amparo M.**
  **Audubon, PA 19403 (US)**
- **CALLAHAN, James, Francis**
  **Philadelphia, PA 19119 (US)**
- **BHATNAGAR, Pradip, Kumar**
  **Exton, PA 19341 (US)**
- **DEL MAR, Eric, G.**
  **Salt Lake City, UT 84108 (US)**
- **BRYAN, William, M.**
  **Phoenixville, PA 19460 (US)**
- **BURGESS, Joelle, L.**
  **Trappe, PA 19426 (US)**

(74) Representative: **Billson, Siân Catherine**
**GlaxoSmithKline**
**Corporate Intellectual Property (CN9.25.1)**
**980 Great West Road**
**Brentford, Middlesex TW8 9GS (GB)**

(56) References cited:
**WO-A-01/08673**
**WO-A-98/45255**
**WO-A-99/51569**
**WO-A1-97/37967**
**WO-A1-99/51241**
**WO-A1-99/51569**
**US-A- 4 645 678**

- **MANGNUS, E. M. ET AL: "Structural modifications of strigol analogs. Influence of the B and C rings on the bioactivity of the germination stimulant GR24" JOURNAL OF AGRICULTURAL AND FOOD CHEMISTRY , 40(7), 1222-9 CODEN: JAFCAU; ISSN: 0021-8561, 1992, XP002336708**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

## FIELD OF INVENTION

The present invention relates to novel calcilytic compounds, pharmaceutical compositions containing these compounds and their use as calcium receptor antagonists.

In mammals, extracellular $Ca^{2+}$ is under rigid homeostatic control and regulates various processes such as blood clotting, nerve and muscle excitability, and proper bone formation. Extracellular $Ca^{2+}$ inhibits the secretion of parathyroid hormone ("PTH") from parathyroid cells, inhibits bone resorption by osteoclasts and stimulates secretion of calcitonin from C-cells. Calcium receptor proteins enable certain specialized cells to respond to changes in extracellular $Ca^{2+}$ concentration.

PTH is the principal endocrine factor regulating $Ca^{2+}$ homeostasis in the blood and extracellular fluids. PTH, by acting on bone and kidney cells, increases the level of $Ca^{2+}$ in the blood. This increase in extracellular $Ca^{2+}$ then acts as a negative feedback signal, depressing PTH secretion. The reciprocal relationship between extracellular $Ca^{2+}$ and PTH secretion forms an important mechanism maintaining bodily $Ca^{2+}$ homeostasis.

Extracellular $Ca^{2+}$ acts directly on parathyroid cells to regulate PTH secretion. The existence of a parathyroid cell surface protein which detects changes in extracellular $Ca^{2+}$ has been confirmed. See Brown et al., Nature 366:574, 1999. In parathyroid cells, this protein, the calcium receptor, acts as a receptor for extracellular $Ca^{2+}$, detects changes in the ion concentration of extracellular $Ca^{2+}$ and initiates a functional cellular response, PTH secretion.

Extracellular $Ca^{2+}$ influences various cell functions, reviewed in Nemeth et al., Cell Calcium 11:319, 1990. For example, extracellular $Ca^{2+}$ plays a role in parafollicular (C-cells) and parathyroid cells. See Nemeth, Cell Calcium 11: 323, 1990. The role of extracellular $Ca^{2+}$ on bone osteoclasts has also been studied. See Zaidi, Bioscience Reports 10: 493, 1990.

Various compounds are known to mimic the effects of extra-cellular $Ca^{2+}$ on a calcium receptor molecule. Calcilytics are compounds able to inhibit calcium receptor activity, thereby causing a decrease in one or more calcium receptor activities evoked by extracellular $Ca^{2+}$. Calcilytics are useful as lead molecules n the discovery, development, design, modification and/or construction of useful calcium modulators, which are active at $Ca^{2+}$ receptors. Such calcilytics are useful in the treatment of various disease states characterized by abnormal levels of one or more components, e.g., polypeptides such as hormones, enzymes or growth factors, the expression and/or secretion of which is regulated or affected by activity at one or more $Ca^{2+}$ receptors. Target diseases or disorders for calcilytic compounds inch de diseases involving abnormal bone and mineral homeostasis.

Abnormal calcium homeostasis is characterized by one or more of the following activities: an abnormal increase or decrease in serum calcium; an abnormal increase or decrease in urinary excretion of calcium; an abnormal increase or decrease in bone calcium levels (for example, as assessed by bone mineral density measurements); an abnormal absorption of dietary calcium; an abnormal increase or decrease in the production and/or release of messengers which affect serum calcium levels such as PTH and calcitonin; and an abnormal change in the response elicited by messengers which affect serum calcium levels.

Thus, calcium receptor antagonists offer a unique approach towards the pharmacotherapy of diseases associated with abnormal bone or mineral homeostasis, such as hypoparathyroidism, osteosarcoma, periodontal disease, fracture healing, osteoarthritis, rheumatoid arthritis, Paget's disease, humoral hypercalcemia associated with malignancy and fracture healing, and osteoporosis.

WO 99/51569 discloses calcium receptor antagonist of Formula (I)

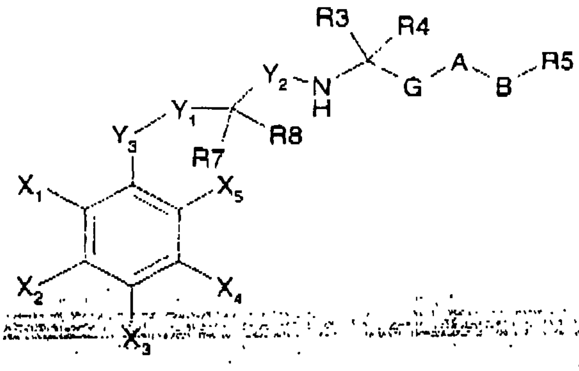

Formula (I)

wherein:

$Y_1$ is a covalent bond, alkylene or alkenylene of up to 4 carbon atoms, unsubstituted or substituted by $C_{1-4}$ alkyl, or O;
$Y_2$ is methylene, unsubstituted or substituted by $C_{1-4}$ alkyl or haloalkyl;
$Y_3$ is covalent bond or O, S, N-$R^{IV}$ or $C_{1-4}$ alkylene-O, $C_{1-4}$ alkylene-S, $C_{1-4}$ alkylene-N-$R^{IV}$;
$R_3$ and $R_4$ are, independently, methyl or ethyl, or, together, form cyclopropyl;
$R_5$ is aryl or fused aryl, dihydro or tetrahydro fused aryl, unsubstituted or substituted with any substituents being selected from the group consisting of OH, halogen, $C_{1-4}$ alkyl $C_{1-4}$ alkoxy, $C_{3-6}$ cycloalkyl, $OSO_2R^{IV}$, CN, $NO_2$, $OCF_3$, $CF_3$, $CH_2CF_3$, $(CH_2)_n$ $CO_2R^{IV}$, and O-$(CH_2)_n$ $CO_2R^{IV}$, wherein n is an integer from 0 to 3 and $R^{IV}$ is selected from the group consisting of H, $C_{1-4}$ alkyl, $C_{3-6}$ cycloalkyl;
or $R_5$ is heteroaryl or fused heteroaryl; wherein the hetero-ring contains N, O or S, and is aromatic, dihydro or tetrahydro, unsubstituted or substituted with any substituents being selected from the group consisting of OH, $OCH_3$, $CH(CH_3)_2$, halogen, $C_{1-4}$ alkyl, $C_{1-4}$ alkoxy, $C_{3-6}$ cycloalkyl, $OSO_2R^{IV}$, CN, $NO_2$, $OCF_3$, $CF_3$, $CH_2CF_3$, $(CH_2)_n$ $CO_2H$, $(CH_2)_n$ $CO_2R^{IV}$, and O-$(CH_2)_n$ $CO_2R^{IV}$;
G is a covalent bond, $CHR_6$ or C-$R_6$, wherein $R_6$ is H, OH or O (forming a ketone);
$R_7$ is H, OH, or O-$C_{1-4}$ alkyl;
$R_8$ is H or $C_{1-4}$ alkyl; or $R_7$ and $R_8$ together form a ketone;
A and B are, independently, selected from the group consisting of a bond, $CH_2$. NH, O, S and C=O. provided that either A or B is selected from $CH_2$ and NH; or A and B together form a bond; or the A-B moiety is represented by CH=CH or C≡C;
wherein
$X_1$ and $X_5$ are independently selected from the group consisting of H, halogen, CN, $NO_2$, $C_{1-4}$ alkyl, cycloalkyl, $CH_2$-aryl, and $CH_2$-heteroaryl; provided that either $X_1$ or $X_5$ is H;
$X_2$, $X_3$ and $X_4$ are selected from the group consisting of H, halogen, O-$C_{1-4}$ alkyl, O-aryl, O-heteroaryl, $CH_2$-aryl, $CH_2$-heteroaryl, alkyl, C(O)aryl, C(O)heteroaryl, CH(OH)aryl, CH(OH)heteroaryl; and J-K;
J is a covalent bond, alkylene, O-alkylene or alkenylene of up to 5 carbon atoms, unsubstituted or substituted by a substituent selected from the group consisting of $C_{1-4}$ alkyl, OH, O(forming a ketone), aryl, heteroaryl, and NR'R'', wherein R' and R" are independently selected from the group consisting of H, alkyl, aryl, heteroaryl C(O) alkyl, C (O)aryl, and C(O)heteroaryl;
K is selected from the group consisting of, $CO_2R^{IV}$, OH, and CN;
and pharmaceutically acceptable salts and complexes thereof.

WO 99/51241 discloses calcium receptor antagonist of Formula (I)

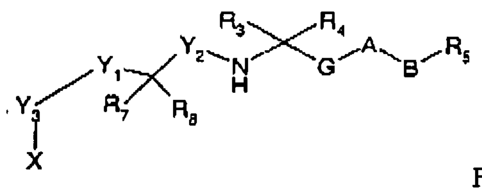

Formula (I)

wherein:

$Y_1$ is a covalent bond, alkylene or alkenylene of up to 4 carbon atoms, unsubstituted or substituted by $C_{1-4}$ alkyl or O;
$Y_2$ is methylene, unsubstituted or substituted by $C_{1-4}$ alkyl or haloalkyl;
$Y_3$ is covalent bond or O, S, N-$R^{IV}$ or $C_{1-4}$ alkylene-O, $C_{1-4}$ alkylene-S, $C_{1-4}$ alkylene-N-$R^{IV}$ ;
$R^{IV}$ is selected from the group consisting of H, $C_{1-4}$ alkyl, $C_{3-6}$ cycloalkyl;
$R_3$ and $R_4$ are, independently, ethyl or ethyl, or, together, form cyclopropyl;
$R_5$ is heteroaryl or fused heteroaryl; wherein the hetero-ring contains N, O or S, and is aromatic, dihydro or tetrahydro, unsubstituted or substituted with any substituents being selected from the group consisting of OH, $OCH_3$, $CH(CH_3)_2$, halogen, $C_{1-4}$ alkyl, $C_{1-4}$ alkoxy, $C_{3-6}$ cycloalkyl, $OSO_2R^{IV}$, CN, $NO_2$, $OCF_3$, $CF_3$, $CH_2CF_3$, $(CH_2)_n$ $CO_2H$, $(CH_2)_n$ $CO_2R^{IV}$, and O-$(CH_2)_n$ $CO_2R^{IV}$; n is an integer from 0 to 3;
G is a covalent bond, $CHR_6$ or C-$R_6$, wherein $R_6$ is H, OH or O (forming a ketone):
$R_7$ is H, OH, or O-$C_{1-4}$ alkyl;
$R_8$ is H or $C_{1-4}$ alkyl; or $R_7$ and $R_8$ together form a ketone;
A and B are, independently, selected from the group consisting of a bond, $CH_2$, NH, O, S and C=O, provided that either A or B is selected from $CH_2$ and NH, or A and B together form a bond; or the A-B moiety is represented by CH=CH or C≡C;
X is selected from sub formulas (Ia) to (Ie) hereinbelow:

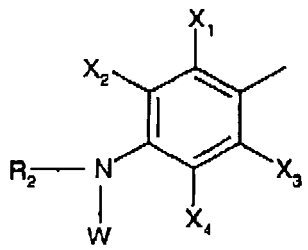

(Ia)

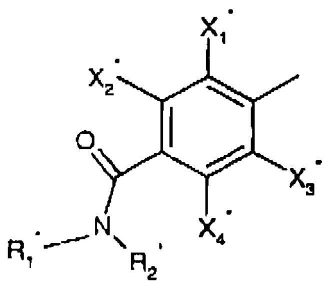

(Ib)

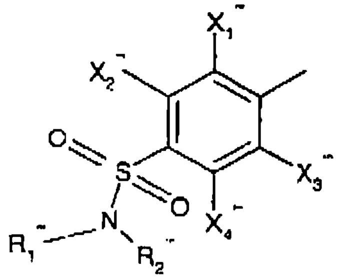

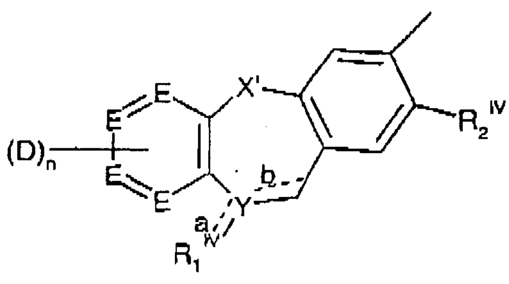

(Id)

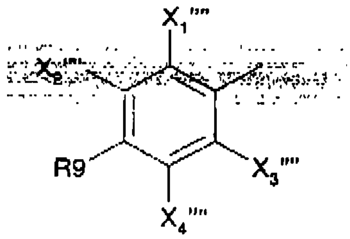

(1e)

wherein

W is selected from the group consisting of $R_1$, $SO_2R_1$, $C(O)R_1$, $SO_2NR_1R_1'$, $C(O)NR_1R_1'$, $C(O)OR_1$, $SO_3R_1'$, wherein $R_1$ and $R_{1'}$ are independently selected from the group consisting of hydrogen, $C_{1-4}$ alkyl, $C_{3-6}$ cycloalkyl, $C_{2-5}$ alkenyl $C_{2-5}$ alkynyl, heterocycloalkyl, aryl and aryl $C_{1-4}$ alkyl; or $R_1$ and $R_1'$ together form a 3 to 7 membered optionally substituted heterocyclic ring; wherein any substituents are selected from the group consisting of CN, aryl, $CO_2R$, $CO_2NHR$, OH, OR, $NH_2$, halo, $CF_3$, $OCF_3$ and $NO_2$; wherein R represents $C_{1-4}$ alkyl, or $C_{3-6}$ cycloalkyl;

$X_1$ is selected from the group consisting of CN, $NO_2$, Cl, F, Br, I, H, R', OR', $CF_3$, $OCF_3$ and $OSO_2R'$, wherein R' represents $C_{1-4}$ alkyl, or $C_{3-6}$ cycloalkyl;

$X_2$, $X_3$ and $X_4$ are, independently, selected from the group consisting of CN, $NO_2$, Cl, F, Br, I, H, R", OR", $CF_3$, $OCF_3$ and $OSO_2R"$, provided that either $X_1$ or $X_3$ is H, wherein R" is $C_{1-4}$ alkyl or haloalkyl; or $X_1$ and $X_2$ together form an aryl or heteroaryl ring, substituted or unsubstituted; wherein the heteroatom is selected from N, S and O; and any substituents are selected from the group consisting of halo, $C_{1-4}$ alkyl, $OCF_3$, $CF_3$, OMe, CN, $OSO_2R'$ and $NO_2$; or $X_3$ and $X_4$ independently represent $C(O)R_{1;}$ and

$R_2$ is selected from the group consisting of hydrogen, $C_{1-4}$ alkyl, $C_{3-6}$ cycloalkyl. $C_{2-5}$ alkenyl, $C_{2-5}$ alkynyl, heterocycloalkyl aryl and aryl-$C_{1-4}$ alkyl;

$X_1"$ is selected from the group consisting of CN, $NO_2$, Cl. F, Br, I, H, R, OR, $CF_3$. $OCF_3$ and $OSO_2R$, wherein R represents $C_{1-4}$ alkyl, or $C_{3-6}$ cycloalkyl;

$X_2"$, $X_3"'$ and $X_4"$ are, independently, selected from the group consisting of CN, $NO_2$, Cl, F, Br, I, H, R', OR', $CF_3$, $OCF_3$ and $OSO_2R'$, provided that either $X"_1$ or

$X"_3$ is H, wherein R' is $C_{1-4}$ alkyl or haloalkyl; or $X_1"$ and $X_2"$ together form an aryl or heteroaryl ring, substituted or unsubstituted; wherein the heteroatom is Selected from N, S and O and any substituents are selected from the group consisting of halo, $C_{1-4}$ alkyl, $OCF_3$, $CF_3$, OMe, CN, $OSO_2$-$C_{1-4}$ alkyl, $OSO_2$-$C_{3-6}$ cycloalkyl and $NO_2$;

or $X_3"$ and $X_4"$ independently represent $C(O)R_1$ ; and

$R_1"$ and $R_2"$ are, independently, selected from the group consisting of hydrogen,

$C_{1-4}$ alkyl, $C_{3-6}$ cycloalkyl, $C_{2-5}$ alkenyl, $C_{2-5}$ alkynyl, heterocycloalkyl and aryl; or $R_1"$ and $R_2"$ together form a 3 to 7 membered optionally substituted heterocyclic ring; wherein any substituents are selected from the group consisting of CN, aryl, $CO_2R"$, $CO_2NHR"$, OH, OR", $NH_2$, halo, $CF_3$, $OCF_3$ and $NO_2$;

wherein R" represents $C_{1-4}$ alkyl, or $C_{3-6}$ cycloalkyl;

$X_1"'$ is selected from the group consisting of CN, $NO_2$, Cl, F, Br, I, H, R, OR, $CF_3$, $OCF_3$ and $OSO_2R$, wherein R represents $C_{1-4}$ alkyl, or $C_{3-6}$ cycloalkyl;

$X_2"'$, $X_3"'$, and $X_4"'$ are, independently, selected from the group consisting of CN, $NO_2$, Cl, F, Br, I, H, R', OR', $CF_3$, $OCF_3$ and $OSO_2R'$, provided that either $X"'_1$ or

$X"'_3$ is H, wherein R' is $C_{1-4}$ alkyl or haloalkyl;

or $X_1'"$ and $X_2"'$ together form an aryl or heteroaryl ring, substituted or unsubstituted; wherein the heteroatom is selected from N, S and O and the substituents are selected from the group consisting of halo, $C_{1-4}$ alkyl, $OCF_3$, $CF_3$, OMe, CN, $OSO_2$-$C_{1-4}$ alkyl, $OSO_2$-$C_{3-6}$ cycloalkyl and $NO_2$;

or $X_3"'$ and $X_4"'$ independently represent $C(O)R_1$ ;

$R_1"'$ and $R_2"'$ are, independently, selected from the group consisting of hydrogen, $C_{1-4}$ alkyl, $C_{3-6}$ cycloalkyl, $C_{2-5}$ alkenyl, $C_{2-5}$ alkynyl, heterocycloalkyl and aryl;

or $R_1"'$ and $R_2"'$ together form a 3 to 7 membered optionally substituted heterocyclic ring; wherein the substituents are selected from the group consisting of CN, aryl, $CO_2R"$, $CO_2NHR"$, OH, OR", $NH_2$, halo, $CF_3$, $OCF_3$ and $NO_2$; wherein R" represents $C_{1-4}$ alkyl, or $C_{3-6}$ cycloalkyl;

D is selected from the group consisting of H, C.N, $NO_2$, Cl, F, Br, I, R, OR, SR, $CF_3$, $OCF_3$ and $OSO_2R$, wherein R represents $C_{1-4}$ alkyl, $C_{3-6}$ cycloalkyl, or $C_{1-10}$ aryl or heteroaryl wherein the heteroatom is selected from N, S and O and substituents are selected from the group consisting of halo, $C_{1-4}$ alkyl, $OCF_3$, $CF_3$, OMe, CN, $OSO_2$-$C_{1-4}$ alkyl, $OSO_2$-$C_{3-6}$ cycloalkyl and $NO_2$;

n is the integer 1 or 2;
each E is independently C or N, provided that no more than two E moieties are N; further provided that when n is 2, each E is C;
a and b are optionally present bonds;
$R_1^{IV}$ is selected from the group consisting of $(CH_2)_nCO_2R'$, $(CH_2)_nCO_2H$, $(CH_2)_nCONR_2$, $(CH_2)_nCH_2OR'$, OR', SR', CN, $NO_2$, Cl, F, Br, I, H, $CF_3$, $OCF_3$, $OSO_2R'$, R' and H; wherein R' represents $C_{1-4}$ alkyl, or $C_{3-6}$ cycloalkyl;
or $R_1^{IV}$ is O, forming a ketone such that Y $R_1^{IV}$ represents -C=O;
$R_2^{IV}$ is selected from the group consisting of hydrogen, CN, $NO_2$ Cl, F, Br, I, H, R'', OR'', $CF_3$, $OCF_3$, and $OSO_2R''$; wherein R'' represents $C_{1-4}$ alkyl, or $C_{3-6}$ cycloalkyl.
Y is selected from the group consisting of C, CH, O, N and S; provided that when
Y is S, $R^{IV}$ is O or not present; further provided that when Y is O, $R_1^{IV}$ is not present;
X' is selected from the group consisting of $CH_2$, NH, O and S. $R_9$ is selected from the group consisting of O-alkyl, O-$CH_2$-aryl, and O- aryl;
$X_1''''$ is selected from the group consisting of CN, $NO_2$, Cl, F, Br, I, H, R, OR, $CF_3$, $OCF_3$ and $OSO_2R$, wherein R represents $C_{1-4}$ alkyl, or $C_{3-6}$ cycloalkyl;
$X_2''''$, $X_3''''$, and $X_4''''$ are, independently, selected from the group consisting of CN, $NO_2$, Cl, F, Br, I, H, R', OR'. $CF_3$, $OCF_3$ and $OSO_2R'$, provided that either $X'''_1$ or
$X''''_3$ is H, wherein R' is $C_{1-4}$ alkyl or haloalkyl;
or $X_1''''$ and $X_2''''$ together form an aryl or heteroaryl ring, substituted or unsubstituted; wherein the heteroatom is selected from N, S and O and the substituents are selected from the group consisting of halo, $C_{1-4}$ alkyl, $OCF_3$, $CF_3$, OMe, CN, $OSO_2$-$C_{1-4}$ alkyl, $OSO_2$-$C_{3-6}$ cycloalkyl and $NO_2$;
or $X_2''''$ and $X_4''''$ independently represent $C(O)R_1$;
and pharmaceutically acceptable salts and complexes thereof.

WO 97/3796 7 discloses compounds able to inhibit calcium receptor activity of structure I

STRUCTURE I

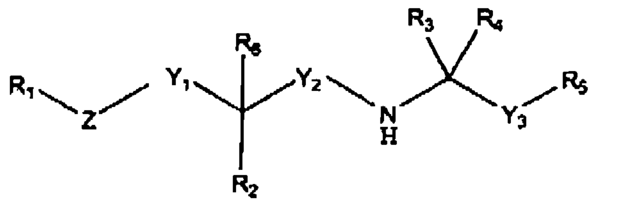

where $R_1$ is selected from the group consisting of : aryl, longer-length alk, and cycloalk ;
$R_3$ is selected from the group consisting of: lower alk, cycloalk, alkoxy, H, OH, =O, C(O) OH, C(O) O-lower alk, C(O) NH-lower alk, C(O)N(lower alk)$_2$, SH, S-lower alk, $NH_2$, NH-lower alk, and N(lower alk)$_2$ ;
$R_3$ and $R_4$ is each independently lower alk or together cyclopropyl ;
$R_5$ is aryl;
$R_6$ if present is either hydrogen, lower alkyl or lower alkenyl, wherein $R_6$ is not present if $R_2$ is =O ;
$Y_1$ is either covalent bond, alkylene, or alkenylene;
$Y_2$ is alkylene ;
$Y_3$ is alkylene; and
Z is selected from the group consisting of: covalent bond, O, S, NH, N-lower alk, alkylene, alkenylene, and alkynylene, provided that if Z is either O, S, NH, or N-lower alk, then $Y_1$ is not a covalent bond, further provided that $Y_1$ and Z may together be a covalent bond;
and pharmaceutically acceptable salts and complexes thereof.

## SUMMARY OF THE INVENTION

The present invention comprises novel calcium receptor antagonists hereinbelow and their use as calcium receptor antagonists in the treatment of a variety of diseases associated with abnormal bone or mineral homeostasis, including but not limited to hypoparathyroidism, osteosarcoma, periodontal disease, fracture healing, osteoarthritis, rheumatoid arthritis, Paget's disease, humoral hypercalcemia associated with malignancy and fracture healing, and osteoporosis.

The present invention further provides a use of an effective amount of a compound, indicated herein below for

the manufacture of a medicament for antagonizing calcium receptors in an animal, including humans.

The present invention further provides a use of an effective amount of a compound indicated herein below for the manufacture of a medicament for increasing serum parathyroid levels in an animal, including humans.

## DETAILED DESCRIPTION OF THE INVENTION

Compounds of the present invention include:

3-{4-Cyano-3-[(R)-2-hydroxy-3-(2-indan-2-yl-1,1-dimethyl-ethylamino)-propoxy]-phenyl)-propionic ethyl ester;
3-{4-Cyano-3-[(R)-2-hydroxy-3-(2-indan-2-yl-1,1-dimethyl-ethylamino)-propoxy]-phenyl}-propionic acid;
3-{4-Cyano-3-[(R)-2-hydoxy-3-(2-indan-2-yl-1,1-dimethyl-ethylamino)-propoxy]-phenyl}-propionic acid isopropyl ester;
3-{4-Cyano-3-[(R)-2-hydroxy-3-(2-indan-2-yl-1,1-dimethyl-ethylamino)-propoxy]-phenyl}-propionic acid 2-ethoxy ethyl ester;
3-[4-cyano-3-[(R)-2-hydroxy-3-(2-indan-2-yl-dimethyl-ethylamino)-propoxy]-phenyl}-propionic acid 2-methoxy-1-methyl-ethyl ester ;
3-(4-Cyano-3-{(R)-3-[1,1-dimethyl-2-(5,6,7,8-tetrahydro-naphthalen-2-yl)-ethylamino]-2-hydroxy-propoxy}-phenyl)-propionic acid ;
3-(4-Cyano-3-{(R)-3-[1,1-dimethyl-2-(5,6,7,8-tetrahydro-naphthalen-2-yl)-ethylamino]-2-hydroxy-propoxy}-phenyl)-propionic acid ethyl ester ;
3-(3-Cyano-4-1{(R)-3-[1,1-dimethyl-2-(5,6,7,8-tetrahydro-naphthalen-2-yl)-ethylamino]-2-hydroxy-propoxy}-phenyl)-propionic acid;
3-(3-Cyano-4-{(R)-3-[1,1-dimethyl-2-(5,6,7,8-tetrahydro-naphthalen-2-yl)-ethylamino]-2-hydroxy-propoxy}-phenyl)-propionic acid ethyl ester ;
3-{4-Cyano-3-[(R)-2-hydroxy-3-(2-indan-5-yl-1,1-dimethyl-ethylamino)-propoxy]-phenyl)-propionic acid; and
3-{4-Cyano-3-[(R)-2-hydroxy-3-(2-indan-5-yl-1,1-dimethyl-ethylamino)-propoxy]-phenyl}-propionate ethyl ester;
and pharmaceutically acceptable salts and complexes thereof.

Preferred compounds of the present invention include:

3-(4-Cyano-3-[(R)-2-hydroxy-3-(2-indan-2-yl-1,1-dimethyl-ethylamino)-propoxy]-phenyl}-propionic ethyl ester; and
3-{4-Cyano-3-[(R)-2-hydroxy-3-(2-indan-2-yl-1,1-dimethyl-ethylamino)-propoxy]-phenyl} -propionic acid;
and pharmaceutically acceptable salts and complexes thereof.

Pharmaceutically acceptable salts are non-toxic salts in the amounts and concentrations at which they are administered.

Pharmaceutically acceptable salts include acid addition salts such as those containing sulfate, hydrochloride, fumarate, maleate, phosphate, sulfamate, acetate, citrate, lactate, tartrate, methanesulfonate, ethanesulfonate, benzenesulfonate, p-toluenesulfonate, cyclohexylsulfamate and quinate. A preferred salt is a hydrochloride. Pharmaceutically acceptable salts can be obtained from acids such as hydrochloric acid, maleic acid, sulfuric acid, phosphoric acid, sulfamic acid, acetic acid, citric acid, lactic acid, tartaric acid, malonic acid, methanesulfonic acid, ethanesulfonic acid, benzenesulfonic acid, p-toluenesulfonic acid, cyclohexylsulfamic acid, fumaric acid, and quinic acid.

Pharmaceutically acceptable salts also include basic addition salts such as those containing benzathine, chloroprocaine, choline, diethanolamine, ethylenediamine, meglumine, procaine, aluminum, calcium, lithium, magnesium, potassium, sodium, ammonium, alkylamine, and zinc, when acidic functional groups, such as carboxylic acid or phenol are present.

The present invention provides compounds above, which can be prepared using standard techniques. An overall strategy for preparing preferred compounds described herein can be carried out as described in this section. The examples that follow illustrate the synthesis of specific compounds. Using the protocols described herein as a model, one of ordinary skill in the art can readily produce compounds of the present invention.

All reagents and solvents were obtained from commercial vendors. Starting materials (e.g., amines and epoxides) were synthesized using standard techniques and procedures,

The compounds are prepared by the general methods described in Schemes 1-3. In general, a solution of a glycidyl ether (e.g., 7 of Scheme I) and a primary amine (e.g., 2-indan-2-yl-1, 1-dimethyl-ethylamine of Scheme 1) in a solvent such as absolute ethanol, acetonitrile, toluene, THF or any other similar solvent optionally in the presence of a suitable catalyst such as $LiClO_4$ (0.1-2 equiv.) is stirred overnight or longer at reflux. The product (e.g., 8-Scheme 1) is purified by chromatography and/or recrystallization. An acid salt e.g., hydrochloride salt is prepared by treatment of the corresponding free base with the acid (e.g., HC] either in gas phase or 4M dioxane solution), or by any other standard

method. Hydrolysis of the ester under either basic or acidic conditions yields the corresponding acid (e.g., 9-Scheme 1).

A general synthesis of the epoxide with compound 7 as an example is outlined in Scheme 2.

The syntheses of some of the required amines are outlined in Scheme 3.

**Scheme 1**

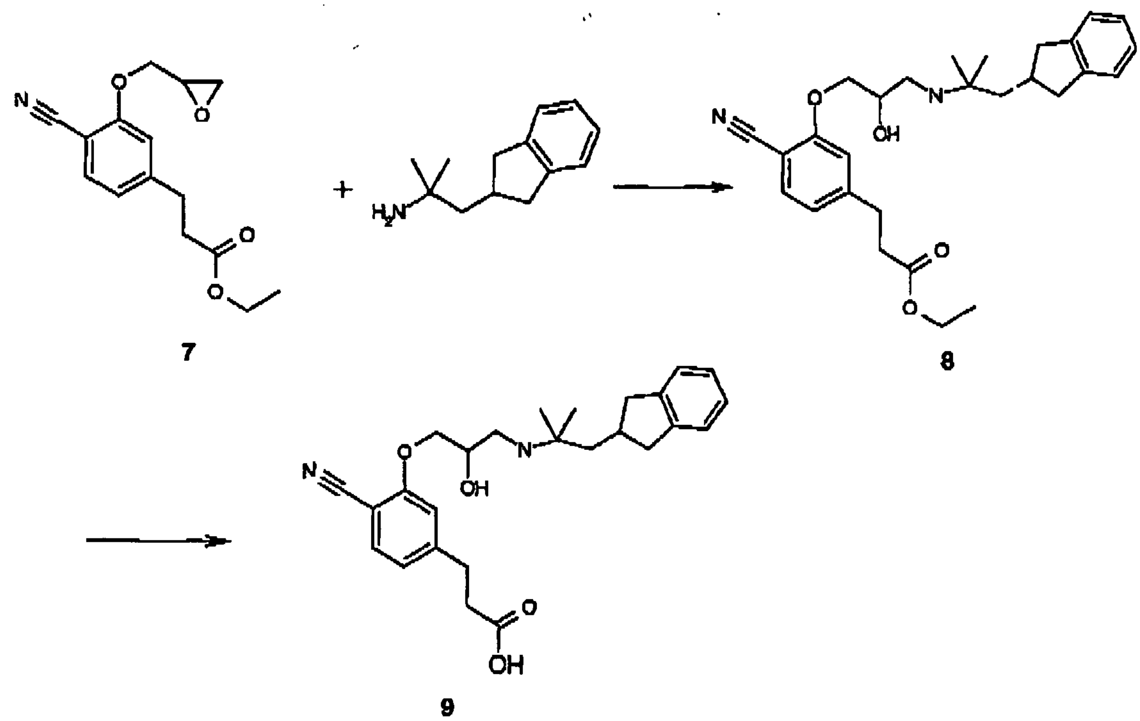

7

8

9

**Scheme 2**

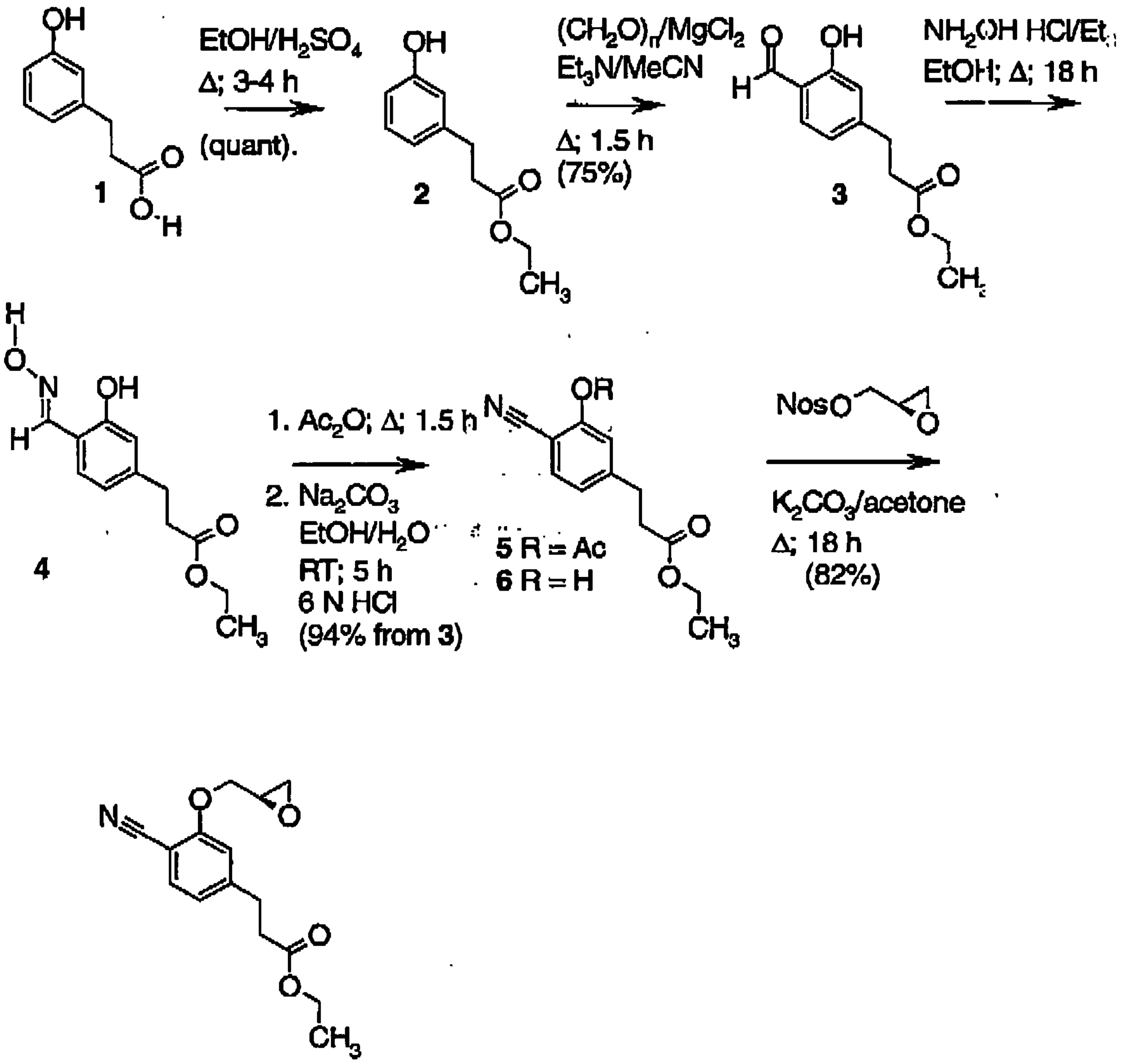
EtOH/$H_2SO_4$
Δ; 3-4 h
(quant).
1
2
$(CH_2O)_n$/$MgCl_2$
$Et_3N$/MeCN
Δ; 1.5 h
(75%)
3
$NH_2OH$ HCl/Et
EtOH; Δ; 18 h
4
1. $Ac_2O$; Δ; 1.5 h
2. $Na_2CO_3$
EtOH/$H_2O$
RT; 5 h
6 N HCl
(94% from 3)
5 R = Ac
6 R = H
K$_2$CO$_3$/acetone
Δ; 18 h
(82%)

7

**Scheme 3**

a)

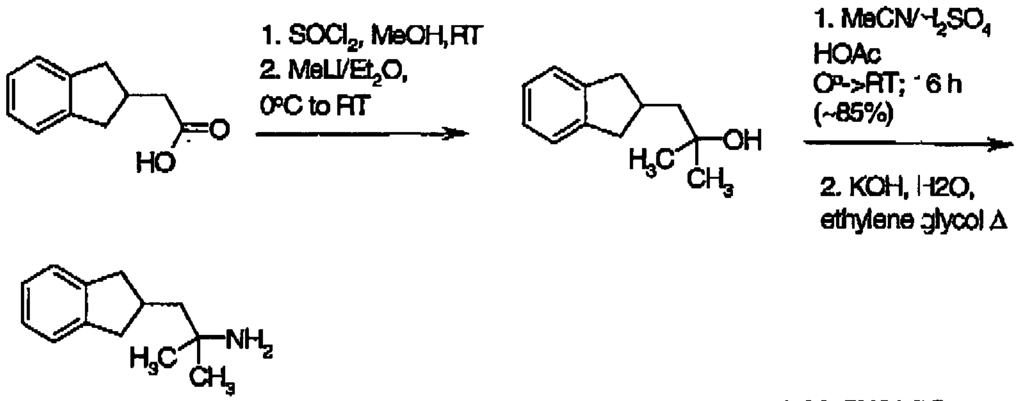


b)

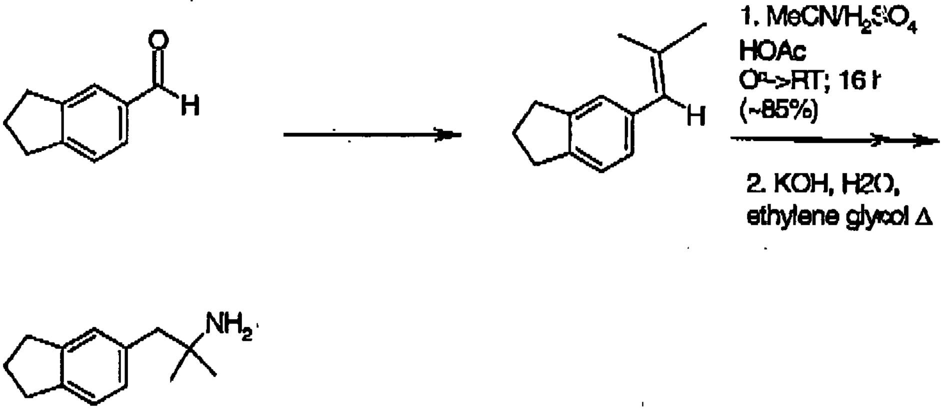


c)

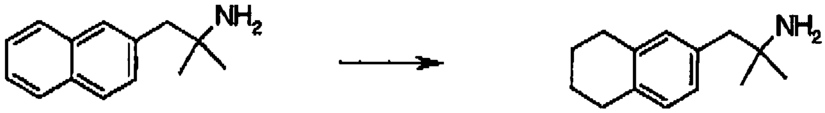

In order to use a compound of the present invention or a pharmaceutically acceptable salt thereof for the treatment of humans and other mammals, it is normally formulated in accordance with standard pharmaceutical practice as a pharmaceutical composition.

The calcilytic compounds can be administered by different routes including intravenous, intraperitoneal, subcutaneous, intramuscular, oral, topical (transdermal), or transmucosal administration. For systemic administration, oral administration is preferred. For oral administration, for example, the compounds can be formulated into conventional oral dosage forms such as capsules, tablets, and liquid preparations such as syrups, elixirs, and concentrated drops.

Alternatively, injection (parenteral administration) may be used, e.g., intramuscular, intravenous, intraperitoneal, and subcutaneous. For injection the compounds of the invention are formulated in liquid solutions, preferably, in physiologically compatible buffers or solutions, such as saline solution, Hunk's solution, or Ringer's solution. In addition, the compounds may be formulated in solid form and redissolved or suspended immediately prior to use. Lyophilized forms can also be produced.

Systemic administration can also be by transmucosal or transdermal means. For transmucosal of transdermal administration, penetrants appropriate to the barrier to be permeated are used in the formulation. Such penetrants are generally known in the art, and include, for example, for transmucosal administration, bile salts and fusidic acid derivatives, In addition, detergents may be used to facilitate permeation. Transmucosal administration, for example, may be through nasal sprays, rectal suppositories, or vaginal suppositories.

For topical administration, the compounds of the invention can be formulated into ointments, salves, gels, or creams, as is generally known in the art.

The amounts of various calcilytic compounds to be administered can be determined by standard procedures

taking into account factors such as the compound $IC_{50}$, $EC_{50}$, the biological half-life of the compound, the age, size and weight of the patient, and the disease or disorder associated with the patient. The importance of these and other factors to be considered are known to those of ordinary skill in the art.

Amounts administered also depend on the routes of administration and the degree of oral bioavailability. For example, for compounds with low oral bioavailability, relatively higher doses will have to be administered.

Preferably, the composition is in unit dosage form. For oral application, for example, a tablet, or capsule may be administered, for nasal application, a metered aerosol dose may be administered, for transdermal application, a topical formulation or patch may be administered and for transmucosal delivery, a buccal patch may be administered. In each case, dosing is such that the patient may administer a single dose.

Each dosage unit for oral administration contains suitably from 0.01 to 50) mg/Kg, and preferably from 0.1 to 50 mg/Kg, of a compound of Formula (1) or a pharmaceutically acceptable salt thereof, calculated as the free base. The daily dosage for parenteral, nasal, oral inhalation, transmucosal or transdermal routes contains suitably from 0.01 mg to 100 mg/Kg, of a compound of Formula(I). A topical formulation contains suitably 0.01 to 5.0% of a compound of Formula (I). The active ingredient may be administered, for example, from 1 to 6 times per day, preferably once, sufficient to exhibit the desired activity, as is readily apparent to one skilled in the art.

As used herein; "treatment" of a disease includes, but is not limited to prevention, retardation and prophylaxis of the disease.

Diseases and disorders which might be treated or prevented, based upon tte affected cells, include bone and mineral-related diseases or disorders; hypoparathyroidism; those of the central nervous system such as seizures, stroke, head trauma, spinal cord injury, hypoxia-induced nerve cell damage, such as occurs in cardiac arrest or neonatal distress, epilepsy, neurodegenerative diseases such as Alzheimer's disease, Huntington's disease and Parkinson's disease, dementia, muscle tension, depression, anxiety, panic disorder, obsessive-compulsive disorder, post-traumatic stress disorder, schizophrenia, neuroleptic malignant syndrome, and Tourette's syndrome; diseases involving excess water reabsorption by the kidney, such as syndrome of inappropriate ADH secretion (SIADH), cirrhosis, congestive heart failure, and nephrosis; hypertension; preventing and/or decreasing renal toxicity from cationic antibiotics (*e.g.*, aminoglycoside antibiotics); gut motility disorders such as diarrhea and spastic colon; GI ulcer diseases; GI diseases with excessive calcium absorption such as sarcoidosis; autoimmune diseases and organ transplant rejection; squamous cell carcinoma; and pancreatitis.

In a preferred embodiment of the present invention, the present compound: are used to increase serum parathyroid hormone ("PTH") levels. Increasing serum PTH levels can be helpful in treating diseases such as hypoparathyroidism, osteosarcoma, periodontal disease, fracture, osteoarthritis, rheumatoid arthritis, Paget's disease, humoral hypercalcemia malignancy and osteoporosis.

Another aspect of the present invention describes a method of treating a patient comprising administering to the patient an amount of a present compound sufficient to increase the serum PTH level. Preferably, the method is carried out by administering an amount of the compound effective to cause an increase in duration and/or quantity of serum PTH level sufficient to have a therapeutic effect.

In various embodiments, the compound administered to a patient causes an increase in serum PTH having a duration of up to one hour, about one to about twenty-four hours, about one to about twelve hours, about one to about six hours; about one to about five hours, about one to about four hours, about two to about five hours, about two to about four hours, or about three to about six hours.

In an alternative embodiment of the present invention, the compound administered to a patient causes an increase in serum PTH having a duration of more than about twenty-four hours provided that it is co-administered with an anti resorptive agent.

In additional different embodiments, the compound administered to a patient causes an increase in serum PTH of up to two fold, two to five fold, five to ten fold, and at least 10 fold, greater than peak serum PTH in the patient. The peak serum level is measured with respect to a patient not undergoing treatment.

In a preferred embodiment of the present invention, the present compound is co-administered with an anti-resorptive agent. Suitable anti-resorptive agents for coadministration include, but are not limited to estrogen, 1, 25 $(OH)_2$ vitamin D3, calcitonin, selective estrogen receptor modulators, vitronectin receptor antagonists V-H+-ATPase inhibitors, src $SH_2$ antagonists, bisphosphonates and cathepsin K inhibitor.

The composition of of the present invention and their pharmaceutically acceptable salts, which are active when given orally, can be formulated as syrups, tablets, capsules and lozenges. A syrup formulation will generally consist of a suspension or solution of the compound or salt in a liquid carrier for example, ethanol, peanut oil, olive oil, glycerine or water with a flavoring or coloring agent. Where the composition is in the form of a tablet, any pharmaceutical carrier routinely used for preparing solid formulations may be used. Examples of such carriers include magnesium stearate, terra alba, talc, gelatin, acacia, stearic acid, starch, lactose and sucrose, Where the composition is in the form of a capsule, any routine encapsulation is suitable, for example using the aforementioned carriers in a hard gelatin capsule shell. Where the composition is in the form of a soft gelatin shell capsule any pharmaceutical carrier routinely used for

preparing dispersions or suspensions may be considered, for example aqueous gums, celluloses, silicates or oils, and are incorporated in a soft gelatin capsule shell.

Typical parenteral compositions consist of a solution or suspension of a compound or salt in a sterile aqueous or non-aqueous carrier optionally containing, a parenterally acceptable oil, for example polyethylene glycol, polyvinylpyrrolidone, lecithin, arachis oil or sesame oil,

Typical compositions for inhalation are in the form of a solution, suspension or emulsion that may be administered as a dry powder or in the form of an aerosol using a conventional propellant such as dichlorodifluoromethane or trichlorofluoromethane.

A typical suppository formulation comprises a compound of the present invention or a pharmaceutically acceptable salt thereof which is active when administered in this way, with a binding and/or lubricating agent, for example polymeric glycols, gelatins, cocoa-butter or other low melting vegetable waxes or fats or their synthetic analogs.

Typical dermal and transdermal formulations comprise a conventional aqueous or non-aqueous vehicle, for example a cream, ointment, lotion or paste or are in the form of a medicated plaster, patch or membrane.

Preferably the composition is in unit dosage form, for example a tablet, capsule or metered aerosol dose, so that the patient may administer a single dose,

No unacceptable toxological effects are expected when compounds of the present invention are administered in accordance with the present invention.

The biological activity of the compounds of Formula (I) are demonstrated by the following tests:

(I) Calcium Receptor Inhibitor Assay

Calcilytic activity was measured by determining the $IC_{50}$ of the test compound for blocking increases of intracellular $Ca^{2+}$ elicited by extracellular $Ca^{2+}$ in HEK 293 4.0-7 cells stably expressing the human calcium receptor. HEK 293 4.0-7 cells were constructed as described by Rogers et al., J. Bone Miner. Res. 10 Suppl. 1:S483, 1995 (hereby incorporated by reference herein). Intracellular $Ca^{2+}$ increases were elicited by increasing extracellular $Ca^{2+}$ from 1 to 1.75 mM. Intracellular $Ca^{2+}$ was measured using fluo-3, a fluorescent Calcium indicator.

The procedure was as follows:

1. Cells were maintained in T-150 flasks in selection media (DMEM supplemented with 10% fetal bovine serum and 200 ug/mL hygromycin B), under 5% $CO_2$:95% air at 37 °C and were grown up to 90% confluency.
2. The medium was decanted and the cell monolayer was washed twice with phosphate-buffered saline (PBS) kept at 37°C. After the second wash, 6 mL of $0.0_2$% EDTA in PBS was added and incubated for 4 minutes at 37 °C. Following the incubation, cells were dispersed by gentle agitation.
3. Cells from 2 or 3 flasks were pooled and pelleted (100 x g). The cellular pellet was resuspended in 10-15 mL of SPF-PCB+ and pelleted again by centrifugation. This washing was done twice.
Sulfate- and phosphate-free parathyroid cell buffer (SPF-PCB) contains 20 mM Na-Hepes, pH 7,4, 126 mM NaCl, 5 mM KCl, and 1 mM $MgCl_2$. SPF-PCB was made up and stored at 4 °C. On the day of use, SPF-PCB was supplemented with 1 mg/mL of D-glucose and 1 mM $CaCl_2$ and then split into two fractions. Tc one fraction, bovine serum albumin (BSA; fraction V, ICN) was added at 5 mg/mL. (SPF-PCB+). This buffer was used for washing, loading and maintaining the cells The BSA-free fraction was used for diluting the cells in the cuvette for measurements of fluorescence.
4. The pellet was resuspended in 10 mL of SPF-PCB+ containing 2.2 uM fluo-3 (Molecular Probes) and incubated at room temperature for 35 minutes.
5. Following the incubation period, the cells were pelleted by centrifugation. The resulting pellet was washed with SPF-PCB+. After this washing, cells were resuspended in SPF-PCB+ at a density of 1-2 x 106 cells/mL.
6. For recording fluorescent signals, 300 uL of cell suspension were diluted in 1.2 mL of SPF buffer containing 1 mM $CaCl_2$ and 1 mg/mL of D-glucose. Measurements of fluorescence were performed at 37 °C with constant stirring using a spectrofluorimeter. Excitation and emission wavelengths were measured at 485 and 535 nm, respectively. To calibrate fluorescence signals, digitonin (5 mg/mL in ethanol) was added to obtain Fmax, and the apparent Fmin was determined by adding Tris-EGTA (2.5 M Tris-Base, 0.3 M EGTA). The concentration of intracellular calcium was calculated using the following equation:

$$\text{Intracellular calcium} = (F\text{-}F_{min}/F_{max}) \times K_d;\ \text{where } K_d \doteq 400\ \text{nM.}$$

7. To determine the potential calcilytic activity of test compounds, cells were incubated with test compound (or vehicle as a control) for 90 seconds before increasing the concentration of extracellular $Ca^{2+}$ from 1 to 2mM. Calcilytic

compounds were detected by their ability to block, in a concentration-dependent manner, increases in the concentration of intracellular $Ca^{2+}$ elicited by extracellular $Ca^{2+}$.

In general, those compounds having lower $IC_{50}$ values in the Calcium Receptor Inhibitor Assay are more preferred compounds, Compounds having an $IC_{50}$ greater than 50 uM were considered to be inactive. Preferred compounds are those having an $IC_{50}$ of 10uM or lower, more preferred compounds have an $IC_{50}$ of 1 uM, and most preferred compounds have an $IC_{50}$ of 0.1 uM or lower.

**(II) Calcium Receptor Binding Assay**

HEK 293 4.0-7 cells stably transfected with the Human Parathyroid Calcium Receptor ("HuPCaR") were scaled up in T180 tissue culture flasks. Plasma membrane is obtained by polytron homogenization or glass douncing in buffer (50 mM Tris-HCl pH 7.4, 1 mM EDTA, 3 mM $MgCl_2$) in the presence of a protease inhibitor cocktail containing 1 uM Leupeptin, 0.04 uM Pepstatin, and 1 mM PMSF. Aliquoted membrane was snap frozen and stored at -80°C. $^3H$ labeled compound was radiolabeled to a radiospecific activity of 44Ci/mmole and was aliquoted and stored in liquid nitrogen for radiochemical stability.

A typical reaction mixture contains 2 nM $^3H$ compound ((R,R)-N-4'-Methoxy-t-3-3'-methyl-1'-ethylphenyl-1-(1-naphthyl)ethylamine), or $^3H$ compounds (R)-N-[2-Hydroxy-3-(3-chloro-2-cyanophenoxy)propyl}-1,1-dimethyl-2-(4-methoxyphenyl)ethylamine 4-10 ug membrane in homogenization buffer containing 0.1% gelatin and 10% EtOH in a reaction volume of 0.5 mL. Incubation is performed in 12 x 75 polyethylene tubes in an ice water bath. To each tube 25 uL of test sample in 100% EtOH is added, followed by 400 uL of cold incubation buffer and 25 uL of 40 nM $^3H$-compound in 100% EtOH.for a final concentration of 2nM. The binding reaction is initiated by the addition, of 50 uL of 80-200 ug/mL HEK 293 4.0-7 membrane diluted in incubation buffer, and allowed to incubate at 4°C for 30 min. Wash buffer is 50 mM Tris-HCl containing 0:1% PEI. Nonspecific binding is determined by the addition of 100-fold excess of unlabeled homologous ligand, and is generally 20% of total binding. The binding reaction is terminated by rapid filtration onto 1% PEI pretreated GF/C filters using a Brandel Harvestor. Filters are placed in scintillation fluid and radioactivity assessed by liquid scintillation counting.

**Examples**

Nuclear magnetic resonance spectra were recorded at either 300 or 400 MHz using, respectively, a Bruker ARX 300 or Bruker AVANCE 400 spectrometer. $CDCl_3$ is deuteriochloroform, DMSO-$d_6$ is hexadeuteriodimethylsulfoxide, and $CD_3OD$ is tetradeuteriomethanol. Chemical shifts are reported in parts per million ($\Delta$) downfield from the internal standard tetramethylsilane. Abbreviations for NMR data are as follows: s=singlet, d=doublet, t=triplet, q=quartet, m=multiplet, dd=doublet of doublets, dt=doublet of triplets, app=apparent br=broad. J indicate the NMR coupling constant measured in Hertz. Fourier transform infrared (FTIR) spectra were recorded on a Nicolet 510 infrared spectrometer. FTIR spectra were recorded in transmission mode, and band positions are reported in inverse wavenumbers ($cm^{-1}$), Mass spectra were taken on either a SCIEX5 or Micromass instruments, using electrospray (ES) ionization techniques, Elemental analyses were obtained using a Perkin-Elmer 240C elemental analyzer. Melting points were taken on a Thomas-Hoover melting point apparatus and are uncorrected. All temperatures are reported in degrees Celsius.

Analtech Silica Gel GF and E. Merck Silica Gel 60 P-254 thin layer plates were used for thin layer chromatography. Both flash and gravity chromatography were carried out on E. Merck Kieselgel 60 (230-400 mesh) silica gel. Analytical and preparative HPLC were carried out on Rainin or Beckman chromatographs. ODS refers to an octadecylsilyl derivatized silica gel chromatographic support. 5 $\mu$ Apex-ODS indicates an octadecylsilyl derivatized silica gel chromatographic support having a nominal particle size of 5 $\mu$, made by Jones Chromatography, Littleton, Colorado. YMC ODS-AQ® is an:ODS chromatographic support and is a registered trademark of YMC Co. Ltd., Kyoto, Japan. PRP-1® is a polymeric (styrenedivinylbenzene) chromatographic support, and is a registered trademark of Hamilton Co., Reno, Nevada) Celite® is a filter aid composed of acid-washed diatomaceous silica, and is a registered trademark of Manville Corp., Denver, Colorado. Following the general procedure described above the following compounds have been synthesized:

**Example 1**

**Preparation of 2-Indan-2-yl-1,1-dimethyl-ethylamine**

**a) Indan-2-yl-acetic acid methyl ester**

A solution of indan-2-yl-acetic acid (Lancaster, 20 g, 0.11 mol) in methanol (200 mL) was stirred and cooled

to 0-10 °C in an ice bath and treated drop-wise w: th thionyl chloride (14.8 g, 0.125 mol). The mixture was stirred at RT for 16 h, concentrated in *vacuo*, and the oily residue was dissolved in ethyl acetate, washed with 2.5 N sodium hydroxide, water, and brine, dried ($MgSO_4$), and concentrated *in vacuo* to give the title compound (21 g, 97%) which solidified.

**b) 1-Indan-2-yl-2-methyl-propen-2-ol**

A solution of the compound from Example 1(a) (6.3 g, 33 mmol) in ether (150 mL) was added drop-wise to 1.4 M methyllithium in ether (100 mL, 4.25 eq) stirred in an ice bath. The mixture was allowed to warm to RT, stirred for 2 h, and very carefully quenched by drop-wise addition of saturated aqueous ammonium chloride (150 mL). The aqueous phase was separated and extracted with ether, and the combined ether phase was washed with brine, dried ($MgSO_4$), and concentrated *in vacuo* to afford the title compound as an oil which crystallized on standing (-89%).

**c) N-(2-Indan-2-yl-1,1-dimethyl-ethyl)-acetamide**

To a mixture of concentrated sulfuric acid (1.7 mL) in acetonitrile (6 mL) stirred in an ice bath for 45 min. a drop-wise solution of the compound from Example 1(b) (3.3 g, 17.3 mmol) in glacial acetic acid (5 mL) was added. The mixture was allowed to warm to RT, stirred for 16 h, poured into ice water, and extracted with ethyl acetate. The combined organic extract was washed with 2.5 N sodium hydroxide, water, and brine, dried ($MgSO_4$), and concentrated in *vacuo* to give an oily residue that was triturated with hexane and a few drops of ethyl acetate, seeded, and cooled to afford a solid which was isolated by filtration to afford the title compound as tan solid (1.9 g, 47%). MS(ES) m/e 231,9 $[M+H]^+$. The filtrate was concentrated in *vacuo* to afford additional title compound as an oil (1.5 g, 37%).

**d) 2-Indan-2-yl-1,1-dimethyl-ethylamine**

A mixture of the compound from Example 1(c) (6.5 g, 28 mmol) in ethylene glycol (170 mL) was treated with crushed potassium hydroxide pellets (13 g), stirred, and heated to 190°C for 24 h. The mixture was poured into water and extracted with ethyl acetate. The combined organic phase was washed with brine and extracted with 1 N hydrochloric acid. The combined acidic extract was washed with ethyl acetate, basified with 2.5 N sodium hydroxide, and extracted with ethyl acetate. The combined organic extract was washed with brine, dried ($MgSO_4$), and concentrated *in vacuo* to afford the title compound (3.2 g, 60%). MS(ES) m/e 190.6 $[M+H]^+$.

**<u>Example 2</u>**

**Preparation of ethyl (R)-4-cyano-3-(oxiranylmethoxy)benzenepropionate**

**a) Ethyl 3-hydroxybenzenepropionate**
A solution of 3-(3-hydroxyphenyl)propionic acid (Lancaster, 66.4 g, 0.4 mol) in ethanol (700 mL) was treated with concentrated sulfuric acid (6 mL), heated to reflux for 2 h, and allowed to cool to RT. The mixture was cooled in ice, neutralized with 10% aqueous sodium carbonate and concentrated *in vacuo* to about 50 mL. Water (-200 mL) was added and the mixture was extracted three-times with ethyl acetate. The combined ethyl acetate extract was washed with water and brine, dried ($Na_2SO_4$), filtered, and concentrated in *vacuo* to yield the title compound as an oil (70 g, 90%).

**b) Ethyl 4-formyl-3-hydroxybenzenepropionate** To a solution of the compound from Example 2(a) (77.2 g; 0.4 mol) in dry acetonitrile (1 L) stirred under argon was added triethylamine (152 g, 1.5 mol) followed by magnesium chloride (57.1 g, 0.6 mol). After stirring for 5 min, paraformaldehyde (81 g) was added and the reaction was refluxed under argon for 1,5 h. The reaction was cooled, 6 N hydrochloric acid (400 mL) was added and the resulting mixture was extracted with ethyl acetate. The combined ethyl acetate extract was washed with water, dried ($MgSO_4$), filtered and concentrated in *vacuo.* The residual oil was purified by flash column chromatography (silica gel, 10% ethyl acetate/hexane) to give the title compound (66.6 g, 75%).

**c) Ethyl 3-hydroxy-4-[(hydroxyimino)methyl]benzenepropionate** A solution of the compound from Example 2 (b) (66.6 g, 0.3 mol) in absolute ethanol (500 mL) was treated with triethylamine (40.4 g, 0.4 mol) followed by hydroxylamine hydrochloride (23 g, 0.33 mol). The reaction was stirred under argon at reflux for 18 h, concentrated *in vacuo,* and the residual oil was dissolved in ethyl acetate and washed with 1N hydrochloric acid. The ethyl acetate phase was dried ($MgSO_4$), filtered, and concentrated in *vacuo* to give the title compound as an oil which was used

in the next step.

**d) Ethyl 3-acetoxy-4cyanobenzenepropionate** The compound from Example 2c) was treated with acetic anhydride (500 mL) and refluxed under argon for 90 min. The reaction was concentrated in *vacuo* and the resulting oil was dissolved in ethyl acetate and washed with water. The ethyl acetate layer was dried ($MgSO_4$), filtered, and concentrated in *vacuo* to give the title compound as an oil which was used in the next step.

**e) Ethyl 4-cyano-3-hydroxybenzenepropionate** A solution of the compound from Example 2(d) was dissolved in ethanol (200 mL) and treated with a solution of sodium carbonate (64 g, 0.6 mol) in water (1.5 L). After stirring at RT for 5 h, the mixture was neutralised with 6 N hydrochloric acid to pH 5 and concentrated in *vacuo.* The resulting mixture was extracted with ethyl acetate. The ethyl acetate solution was dried ($MgSO_4$), filtered, and concentrated in *vacuo* to give the title compound as an oil [61.9 g, 94.2% overall yield from the compound of Preparation 2(c)].

**f) Ethyl (R)-4-cyano-3-(oxiranylmethoxy)benzenepropionate** A solution of the compound from Example 2(e) (28.3 g, 0.13 mol) and (2R)-glycidyl 3-nitrobenzenesulfonate (33.7 g, 0.13 mol) in dry acetone (500 mL) was treated with potassium carbonate (36 g, 0.26 mol) and refluxed under argon for 18 h. The reaction was cooled, filtered, and the filtrate was concentrated *in vacua* and the residue was purified by flash column chromatography (silica, 30% ethyl acetate/hexane) to yield the title compound (29.5 g, 82.4%).

## Example 3

**Preparation of Ethyl 3-(4-Cyano->[(R)-2-hydroxy-3-(2-indan-2-yl-1,1-dimethyl-ethylamino)-propoxy]-phenyl)-propionate**

A mixture the compounds from Example 2(f) (6 g, 31.7 mmol) and Example 1(d) (8.6 g, 31,7 mmol) in absolute ethanol (200 mL) was stirred and heated to reflux for 56 h, cooled, concentrated *in vacuo.* The residue was dissolved in dichloromethane (30 mL) and acidified with 1.0 N hydrogen chloride in ether. The white solid which formed was isolated by filtration and recrystallized to afford the title compound (10 g, 63%). mp (dichloromethane/ether) 155-157°C; MS(ES) m/e 465.4 $[M+H]^+$.

## Example 4

**Preparation of 3-{4-Cyano-3-[(R)-2-hydroxy-3-(2-indan-2-yl-1,1-dimethylethylamino)-propoxy]-phenyl}-propionic acid**

A solution of the compound from Example 3 (1.5 g, 3 mmol) in ethanol (120 mL) and water (40 mL) was treated with 2.5 N sodium hydroxide (5 mL) and stirred for RT under argon overnight. The ethanol was removed *in vacuo,* and the pH was adjusted to pH 5 with 1 N hydrochloric acid while stirring. The precipitated white solid was collected by filtration, washed with water and dried in *vacuo* to afford the title compound (1.3 g, quant). MS(ES) m/e 437.4 $[M+H]^+$.

## Example 5

**Preparation of 3-{4-Cyano-3-[(R)-2-hydroxy-3-(2-indan-2-yl-1,1-dimethylethylamino)-propoxy-phenyl)-propionic add hydrochloride**

The compound from Example 4 (80 mg, 0.18 mmol) was suspended in acetonitrile (8 mL) and treated with 1 N hydrogen chloride in ether dropwise with stirring until the solid dissolved. The mixture was quickly filtered and cooled in an ice bath. The white crystalline solid which formed was isolated by filtration, washed with ether, and dried in vacuo to afford the title compound (80 mg, 94%). Mp 180-181°C; MS(ES) m/e 437.2 $[M+H]^+$.

## Example 6

**Preparation of 3-14-Cyano-3-[(R)-2-hydroxy-3-(2-indan-2-yl-1,1-dimethylethylamino)-propoxyl]-phenyl)-propionic acid isopropyl ester trifluoroacetate salt**

**a) 3-(4-Cyano-3-hydroxy-phenyl)-propionic acid**

A solution of 3-(4-Cyano-3-hydroxy-phenyl)-propionic acid ethyl ester (2.2 g, 10 mmol) in ethanol (10 mL) and

water (40 mL) was treated with aqueous sodium hydroxide solution (2.5 M, 44 mL) at room temperature overnight. The solvent was removed under vacuum and the residue was dissolved in water (150 mL). The pH of the aqueous solution was adjusted to 2-4 by the addition of 3N HCl (aqueous) and the precipitate was then filtered and washed with water to give the title compound as white solid (1.88 g, 0.98 mmol, 98.4% yield). ESMS m/z: 192 $[M+H]^+$.

**b) 3-(4-cyano-3-hydroxy-phenyl)-propionic acid isopropyl ester**

A solution of compound from Example 6 (a) (0.25 g, 1.3 mmol) and propan 2-ol (0.4 g, 0.67 mmol) in $CH_2Cl_2$ (10 ml) was treated dropwise with a solution of 4. pyrrolidinopyridine (0.2g, 1.3 mmol) in $CH_2Cl_2$ (1 mL). The reaction mixture under $N_2$ was cooled to 0 °C and then treated with EDC (1-[3-(dimethylamino) propyl]-3-ethylcarbodiimide hydrochloride (Aldirch, 0,27 g, 1,4 mmol). The subsequent mixture was stirred at 0 °C for 30 min and slowly brought to room temperature and stirred overnight. The reaction mixture was washed with 3 N HCl (3x25 mL), brine (1x25 mL) and $H_2O$ (1x25 mL). The organic layer was dried over anhydrous sodium sulfate and concentrated under vacuum to give the above titled compound.

**c) 3-(4-Cyano-3-oxiranylmethoxy-phenyl)-propionic acid isopropyl ester**

A solution of compound from Example 6 (b), R-glycidylnosylate (0.33 g; 1.4 mmol) and $K_2CO_3$ (0,36 g, 2.6 mmol) in acetone (10 mL) was refluxed under $N_2$ overnight. The reaction mixture was then cooled to room temperature and filtered. The filtrate was concentrated under vacuum to give the above titled compound as a light brown oil.

**d) 3-{4-Cyano-3-[(R)-2-hydroxy-3-(2-indan-2-yl-1,1-dimethyl-ethylamino)-propoxyl]-phenyl}propionic acid isopropyl ester trifluoroacetate salt**

The compound from Example 6 (c) and the compound from Example 1 (d) was dissolved in toluene (12.5 mL) and the mixture was stirred at 120° C overnight. The solvent was removed under vacuum and the residue was purified by preparative. HPLC (Gilson) to give the above titled compound as a light yellow oil. ESMS m/z 479 $[M+H]^+$.

**<u>Example 7</u>**

**Preparation of 3-{4-Cyano-3-[(R)-2-hydroxy-9-(2-indan-2-yl-1,1-dimethyl-ethylamino)-propoxy]-phenyl}-prnpionic acid 2-ethoxy ethyl ester trifluoroacetate salt**

The title compound was prepared by the same procedure as Example 6 except that propan-2-ol was replaced with 2-ethoxy-ethanol to give the above titled compound as an oil. ESMS m/z: 509 $[M+H]^+$.

**<u>Example 8</u>**

**Preparation of 3-(3-Cyano-4-[(R)-3-[1,1-dimethyl-2-(5,6,7,8.tetrahydronaphthalen-2-yl)-ethylamino]-2-hydroxy-propoxy}-phenyl)-propionic add**

Following the procedure described in Example 3 using ethyl (R)-2-cyano-4-(oxiranylmethoxy)benzenepropionate and the amine from example 21(a) the above titled compound was obtained, MS(ES) mix 451,4 $(M+H)^+$.

**<u>Example 9</u>**

**3-{4-Cyano-3-[(R)-2-hydroxy-3-(2-indan-5-yl-1,1-dimethyl-ethylamino)propoxy]-phenyl}-propionic acid**

**a) 5-(2-Methyl-propenyl)-indan**

In a flamed dried flask under argon isopropyltriphenylphosphonium iodide (6.48 g, 15 mmol) in 30 mL of dry THF was cooled to 0°C. Then n-BuLi (2.5 M) in hexanes (6.0 mL) was added via syringe. The reaction mixture was stirred 0 °C for 45 min. Then a solution of the 2,3-dihydro-1H-indene-5-carboxaldehyde (J. Med. Chem. 1993, 36, 3700-3706) was added (1.7, 12.0 mmol) in THF (20 mL) was added drop-wise and the reaction was stirred under Argon at RT overnight. The reaction was filtered, the filtrate was concentrated in vacuo and the residue purified by flash colum chromatography (ethyl acetate/hexane, 1:99) to.yield the above titled compound as a pale yellow oil (1.7 g, 67%).

**b) N-(2-Indan-5-yl-1,1-dimethyl-ethyl)-acetamide**

Following the procedure described in Example 1 (c) the above titled compound was obtained as a crystalline solid. Melting point 130-131 °C (ethyl acetate); MS(ES) m/z 463.7 $(2M+H)^+$, 322.7 $(M+H)^+$; Elemental Analysis: theoretical for $C_{15}H_{21}NO$: C, 77.85; H, 915; N, 6.05; found: C, 77.54; H, 9.09; N, 6.03.

**c) 2-Inda-5-yl-1,1-dimethyl-ethylamine**

Following the procedure described in experiment 1 (d), the above titled compound was obtained. MS(ES) m/z 190.6 $(M+H)^+$.

**d) Ethyl 3-(4-Cyano-3-[(R)-2-hydroxy-3-(2-indan-5-yl-1,1-dimethyl-ethylamino)-propoxy]-phenyl}-propionate hydrochloride salt.**

Following the procedure described in Example 3 using the amine from Example 23 (c) the above titled compound was obtained. MS(ES) m/z 465.8 $(M+H)^+$; Elemental analysis: theoretical for $C_{28}H_{36}N_2O_4$.HCl.3/4 $H_2O$: C, 65.34; H, 7.4.4; N, 5.44; found: C, 65,11; H, 7.66; N, 5.25.

**e) 3-{4-Cyano-3-[(R)-2-hydroxy-3-(2-indan-5-yl-1,1-dimethyl-ethylamino)-propoxy]-phenyl}-propionic acid**

Following the procedure described in Example 4 the title compound was obtained. MS(ES) m/z 437.8 $(M+H)^+$.

<u>**Example 24**</u>

<u>**Parental Formulation**</u>

A pharmaceutical composition for parenteral administration is prepared by dissolving an appropriate amount of a compound of the present invention in polyethylene glycol with heating. This solution is then diluted with water for injections (to 100 mL). The solution is then rendered sterile by filtration through a 0.22 micron membrane filter and sealed in sterile containers.

## Claims

**1.** A compound selected from the group consisting of:

3-{4-Cyano-3-[(R)-2-hydroxy-3-(2-indan-2-yl-1,1-dimethyl-ethylamino)-propoxy]-phenyl}-propionic ethyl ester;
3-{4-Cyano-3-[(R)-2-hydroxy-3-(2-indan-2-yl-1,1-dimethyl-ethylamino)-propoxy]-phenyl}-propionic acid;
3-{4-Cyano-3-[(R)-2-hydoxy-3-(2-indan-2-yl-1,1-dimethyl-ethylamino)-propoxyl]-phenyl}-propionic acid isopropyl ester;
3-{4-Cyano-3-[(R)-2-hydroxy-3-(2-indan-2-yl-1,1-dimethyl-ethylamino)-propoxy]-phenyl}-propionic acid 2-ethoxy ethyl ester;
3-{4-cyano-3-[(R)-2-hydroxy-3-(2-indan-2-yl-dimethyl-ethylamino)-propoxy]-phenyl}-propionic acid 2-methoxy-1-methyl-ethyl ester;
3-(4- Cyano- 3- { (R)- 3-[1,1- dimethyl- 2-(5,6,7,8- tetrahydro- naphthalen- 2- yl)-ethylamino]- 2- hydroxy- propoxyl-phenyl)-propionic acid;
3-(4- Cyano- 3- { (R)- 3-[1,1'- dimethyl- 2-(5,6,7,8- tetrahydro- naphthalen- 2- yl)-ethylamino]- 2- hydroxy- propoxy}-phenyl)-propionic acid ethyl ester;
3-(3- Cyano- 4- { (R)- 3-[1,1- dimethyl- 2-(5,6,7,8- tetrahydro- naphthalen- 2- yl)-ethylamino]- 2- hydroxy- propoxy}-phenyl)-propionic acid;
3-(3- Cyano- 4- { (R)- 3-[1,1- dimethyl- 2-(5,6,7,8- tetrahydro- naphthalen- 2- yl)-ethylamino]- 2- hydroxy- propoxy}-phenyl)-propionic acid ethyl ester;
3- {4-Cyano-3-[(R)-2-hydroxy-3-(2-indan-5-yl-1,1-dimethyl-ethylamino)-propoxyphenyl}-propionic acid; and
3-[4-Cyano-3-[(R)-2-hydroxy-3-(2-indan-5-yl-1,1-dimethyl-ethylamino)-propoxyl-phenyl}-propionate ethyl ester;and pharmaceutically acceptable salts and complexes thereof.

**2.** A compound according to claim 1 selected from the group consisting of:

3-{4-Cyano-3-[(R)-2-hydroxy-3-(2-indan-2-yl-1,1-dimethyl-ethylamino)-propoxyphenyl}-propionic ethyl ester; and
3-{4-Cyano-3-[(R)-2-hydroxy-3-(2-indan-2-yl-1,1-dimethyl-ethylamino)-propoxy: - phenyl}-propionic acid;
and pharmaceutically acceptable salts and complexes thereof.

**3.** A pharmaceutical composition comprising the compounds according to claim 1 or 2 or a pharmaceutical acceptable salt or complex thereof.

**4.** A pharmaceutical composition according to claim 3 further comprising a pharmaceutical carrier or diluent thereof.

**5.** Use of a compound according to claim 1 or 2 for the manufacture of a medicament for increasing serum parathyroid levels.

**6.** Use of a compound according to claim 1 or 2 for the manufacture of a medicament for antagonizing a calcium receptor.

**7.** Use of a compound according to claim 1 or 2 for the manufacture of a medicament for treating a disease or disorder **characterized by** an abnormal bone or mineral homeostasis.

**8.** The use according to claim 7 wherein the bone or mineral disease or disorder is selected from the group consisting of osteosarcoma, periodontal disease, fracture healing, osteoarthritis, rheumatoid arthritis, Paget's disease, humoral hypercalcemia, malignancy and osteoporosis.

**9.** The use according to claim 8 wherein the bone or mineral disease or disorder is osteoporosis.

**10.** Use according to any one of claims 5 to 9 wherein the calcilytic compound is co-administered with an anti-resorptive agent.

**11.** The use according to claim 10 wherein the anti-resorptive agent is selected from the group consisting of estrogen, 1, 25 $(OH)_2$ vitamin D3, calcitonin, selective estrogen receptor modulators, vitronectin receptor antagonists, V-H+-ATPase inhibitors, src $SH_2$ antagonists, bisphosphonates and cathepsin K inhibitors,

## Patentansprüche

**1.** Verbindung, ausgewählt aus:

3-{4-Cyano-3-[(R)-2-hydroxy-3-(2-indan-2-yl-1,1-dimethyl-ethylamino)-propoxy]-phenyl}-propionsäureethylester;
3-{4-Cyano-3-[(R)-2-hydroxy-3-(2-indan-2-yl-1,1-dimethyl-ethylamino)-propoxy]-phenyl}-propionsäure;
3-{4-Cyano-3-[(R)-2-hydroxy-3-(2-indan-2-yl-1,1-dimethyl-ethylamino)-propoxy]-phenyl}-propionsäureisopropylester;
3- {4- Cyano- 3-[(R)- 2- hydroxy- 3-(2- indan- 2- yl- 1,1- dimethyl- ethylamino)-propoxy]-phenyl}-propionsäure- 2-ethoxyethylester;
3- {4- Cyano- 3-[(R)- 2- hydroxy- 3-(2- indan- 2- yl- dimethyl- ethylamino)-propoxy]-phenyl}-propionsäure- 2- methoxy-1-methyl-ethylester;
3-(4- Cyano- 3- { (R)- 3-[1,1- dimethyl- 2-(5,6,7,8- tetrahydro- naphthalen- 2- yl)-ethylamino]- 2- hydroxy- propoxy}-phenyl)-propionsäure;
3-(4- Cyano- 3- { (R)- 3-[1,1- dimethyl- 2-(5,6,7,8- tetrahydro- naphthalen- 2- yl)-ethylamino]- 2- hydroxy- propoxy}-phenyl)-propionsäureethylester;
3-(3- Cyano- 4- { (R)- 3-[1,1- dimethyl- 2-(5,6,7,8- tetrahydro- naphthalen- 2- yl)-ethylamino]- 2- hydroxy- propoxy}-phenyl)-propionsäure;
3-(3- Cyano- 4- { (R)- 3-[1,1- dimethyl- 2-(5,6,7,8- tetrahydro- naphthalen- 2- yl)-ethylamino]- 2- hydroxy- propoxy}-phenyl)-propionsäureethylester;
3-{4-Cyano-3-[(R)-2-hydroxy-3-(2-indan-5-yl-1,1-dimethyl-ethylamino)-propoxy]-phenyl}-propionsäure; und
3-{4-Cyano-3-[(R)-2-hydroxy-3-(2-indan-5-yl-1,1-dimethyl-ethylamino)-propoxy]-phenyl}-propionsäureethylester;
und pharmazeutisch verträgliche Salze und Komplexe davon.

2. Verbindung gemäß Anspruch 1, ausgewählt aus:

3-{4-Cyano-3-[(R)-2-hydroxy-3-(2-indan-2-yl-1,1-dimethyl-ethylamino)-propoxyl]-phenyl}-propionsäureethylester; und
3-{4-Cyano-3-[(R)-2-hydroxy-3-(2-indan-2-yl-1,1-dimethyl-ethylamino)-propoxyl]-phenyl}-propionsäure;
und pharmazeutisch verträgliche Salze und Komplexe davon.

3. Arzneimittel, umfassend die Verbindungen gemäß Anspruch 1 oder 2 oder ein pharmazeutisch verträgliches Salz oder ein Komplex davon.

4. Arzneimittel gemäß Anspruch 3, weiterhin umfassend einen pharmazeutischen Träger oder ein Verdünnungsmittel davon.

5. Verwendung einer Verbindung gemäß Anspruch 1 oder 2 zur Herstellung eines Medikaments zur Erhöhung des Serum-Parathormonspiegels.

6. Verwendung einer Verbindung gemäß Anspruch 1 oder 2 zur Herstellung eines Medikaments zum Antagonisieren eines Calciumrezeptors.

7. Verwendung einer Verbindung gemäß Anspruch 1 oder 2 zur Herstellung eines Medikaments zur Behandlung einer Krankheit oder Störung **gekennzeichnet durch** eine abnormale Knochen- oder Mineralhomöostase.

8. Verwendung gemäß Anspruch 7, wobei die Knochen- oder Mineralkrankleit oder -störung ausgewählt ist aus Osteosarkom, Parodontalerkrankungen, Frakturheilung, Osteoarthritis, Rheumatoidarthritis, Morbus Paget, humorale Hyperkalziämie, Malignität und Osteoporose.

9. Verwendung gemäß Anspruch 8, wobei die Knochen- oder Mineralkrankieit oder -störung Osteoporose ist.

10. Verwendung gemäß einem der Ansprüche 5 bis 9, wobei die calcilytische Verbindung mit einem Antiresorptionsmittel zusammen verabreicht wird.

11. Verwendung gemäß Anspruch 10, wobei das Antiresorptionsmittel aus Östrogen, 1,25-Dihydroxyvitamin D3, Calcitonin, selektive Östrogen-Rezeptor-Modulatoren, Vitronektin-Rezeptor-Antagonisten, V-$H^+$-ATPase-Inhibitoren, Src $SH_2$-Antagonisten, Bisphosphonaten und Kathepsin-K-Inhibitoren ausgewählt ist.

## Revendications

1. Composé choisi dans le groupe consistant en :

l'ester éthylique d'acide 3-{4-cyano-3-[(R)-2-hydroxy-3-(2-indane-2-yl-1,1-diméthyl-éthylamino)-propoxy]-phényl)-propionique ;
l'acide 3-{4-cyano-3-[(R)-2-hydroxy-3-(2-indane-2-yl-1,1-diméthyl-éthylamino)-propoxy]-phényl}-propionique ;
l'ester isopropylique d'acide 3-{4-cyano-3-[(R)-2-hydroxy-3-(2-indane-2-yl-1,1-diméthyl-éthylamino)-propoxyl-phennyl)-propionique ;
l'ester 2-éthoxy-éthylique d'acide 3-{4-cyano-3-[(R)-2-hydroxy-3-(2-indane-2-yl-1,1-diméthyl-éthylamino)-propoxy]-phényl}-propionique ;
l'ester 2-méthoxy-1-méthyl-éthylique d'acide 3-{4-cyano-3-[(R)-2-hydroxy-3-(2-indane-2-yl-diméthyl-éthylamino)-propoxy]-phényl}-propionique ;
l'acide 3-(4-cyano-3-{(R)-3-[1,1-diméthyl-2-(5,5,7,8-tétrahydronaphtalène-2-yl)-éthylamino]-2-hydroxy-propoxy}-phényl)-propionique ;
l'ester éthylique d'acide 3-(4-cyano-3-{(R)-3-[1,1-diméthyl-2-(5,6,7,8-tétrahydronaphtalène-2-yl)-éthylanino]-2-hydroxy-propoxy}-phényl)-propionique ;
l'acide 3-(3-cyano-4-{(R)-3-[1,1-diméthyl-2-(5,5,7,8-tétrahydronaphtalène-2-yl)-éthylamino]-2-hydroxy-propoxy}-phényl)-propionique ;
l'ester éthylique d'acide 3-(3-cyano-4-{(R)-3-[1,1-diméthyl-2-(5,6,7,8-tétrahydronaphtalène-2-yl)-éthylanino]-2-hydroxy-propoxy}-phényl)-propionique ;
l'acide 3-{4-cyano-3-[(R)-2-hydroxy-3-(2-indane-5-yl-1,1-diméthyl-éthylamino)-propoxy]-phényl}-propionique ;

et
l'ester éthylique de 3-{4-cyano-3-[(R)-2-hydroxy-3-(2-indane-5-yl-1,1-diméthyl-éthylamino)-propoxy]-phényl}-propionate ; et ses sels et complexes pharmaceutiquement acceptables.

**2.** Composé suivant la revendication 1, choisi dans le groupe consistant en :

l'ester éthylique d'acide 3-{4-cyano-3-[(R)-2-hydroxy-3-(2-indane-2-yl-1,1-diméthyl-éthylamino)-propoxy]-phényl}-propionique ; et
l'acide 3-{4-cyano-3-[(R)-2-hydroxy-3-(2-indane-2-yl-1,1-diméthyl-éthylamino)-propoxy]-phényl}-propionique ; et ses sels et complexes pharmaceutiquement acceptables.

**3.** Composition pharmaceutique comprenant les composés suivant la revendication 1 ou 2 ou un de leurs sels ou complexes pharmaceutiquement acceptables.

**4.** Composition pharmaceutique suivant la revendication 3, comprenant en outre un support ou diluant pharmaceutique approprié.

**5.** Utilisation d'un composé suivant la revendication 1 ou 2 pour la production d'un médicament destiné à augmenter les taux sériques d'hormone parathyroïdienne.

**6.** Utilisation d'un composé suivant la revendication 1 ou 2 pour la production d'un médicament destiné à antagoniser un récepteur du calcium.

**7.** Utilisation d'un composé suivant la revendication 1 ou 2 pour la production d'un médicament destiné au traitement d'une maladie ou d'un trouble **caractérisé par** une homéostase anormale des os ou des substances minérales.

**8.** Utilisation suivant la revendication 7, dans laquelle la maladie ou le trouble des os ou des substances minérales est choisi dans le groupe consistant en un ostéosarcome, une maladie périodontique, la guérison de fractures, l'arthrose, la polyarthrite rhumatoïde, la maladie de Paget, l'hypercalcémie humorale, une affection maligne et l'ostéoporose.

**9.** Utilisation suivant la revendication 8, dans laquelle la maladie ou le trouble des os ou des substances minérales est l'ostéoporose.

**10.** Utilisation suivant l'une quelconque des revendications 5 à 9, dans laquelle le composé calcilytique est coadaministré avec un agent antirésorption.

**11.** Utilisation suivant la revendication 10, dans laquelle l'agent antirésorption est choisi dans le groupe consistant en un estrogène, la 1,25$(OH)_2$vitamine D3, la calcitonine, des modulateurs des récepteurs d'oestrogènes sélectifs, des antagonistes du récepteur de vitronectine, des inhibiteurs de V-H+-ATPase, des antagonistes de src $SH_2$, des bisphosphonates et des inhibiteurs de cathepsine 1C.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9951569 A
- WO 9951241 A
- WO 9737967 A


### Non-patent literature cited in the description


- **BROWN et al.** *Nature,* vol. 366, 574
- **NEMETH et al.** *Cell Calcium,* 1990, vol. 11, 319
- **NEMETH.** *Cell Calcium,* 1990, vol. 11, 323
- **ZAIDI.** *Bioscience Reports,* 1990, vol. 10, 493
- **ROGERS et al.** *J. Bone Miner. Res.,* 1995, vol. 10 (1), 483
- *J. Med. Chem.,* 1993, vol. 36, 3700-3706